# EUROPEAN PATENT APPLICATION

(11) **EP 3 192 798 A1**
(43) Date of publication of application: **19.07.2017**
(21) Application number: 15833877.2
(22) Date of filing: 17.08.2015
(51) Int. Cl.: C07H 17/08, A61K 31/7048, A61P 31/04

(54) **C-4''-SUBSTITUTED MACROLIDE COMPOUND**

(30) Priority: 18.08.2014 JP 2014165848
(71) Applicant: Taisho Pharmaceutical Co., Ltd., Tokyo 170-8633 (JP)
(72) Inventor: SUGIMOTO Tomohiro, Tokyo 170-8633 (JP); HAYASHI Masato, Tokyo 170-8633 (JP); KAWAGUCHI Takanori, Tokyo 170-8633 (JP)
(74) Representative: Hoffmann Eitle
(86) International application number: PCT/JP2015/072993
(87) International publication number: WO 2016/027755

(57) **Abstract**

A useful novel compound that shows superior antibacterial activity also against erythromycin resistant bacteria, for example, resistant pneumococci, streptococci, mycoplasmas, and the like, against which sufficient antibacterial activity cannot be obtained with conventional macrolide antibiotics, or a pharmaceutically acceptable salt thereof, or a hydrate or a solvate thereof.

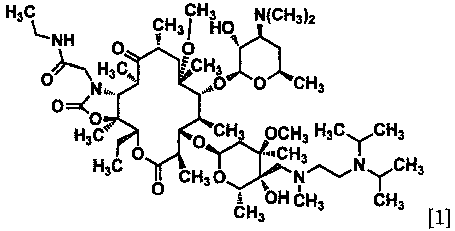

## Description

### Technical Field

The present invention relates to a novel antibiotic having an erythromycin-like structure. More specifically, the present invention relates to a medicament used for prophylactic and/or therapeutic treatment of an infectious disease, which contains a macrolide compound having a methyl group substituted with a substituent having nitrogen atom at the 4"-position of the cladinose as an active ingredient.

### Background Art

Erythromycin A is an antibiotic which has been widely used as a therapeutic agent for infectious diseases caused by Gram-positive bacteria, mycoplasmas, and the like. However, due to decomposition by gastric acid, erythromycin has a drawback of inconstant pharmacokinetics. Therefore, derivatives of erythromycin having increased stability to acids were researched. As a result, macrolides having stable pharmacokinetics such as clarithromycin, azithromycin (Patent documents 1 and 2) and roxithromycin have been developed. These macrolide agents have been applied in a therapeutic field of respiratory infectious diseases of ambulatory patients, and therefore, they are required to have a potent antibacterial activity especially against pneumococci, streptococci, and *Haemophilus influenzae* which are frequently isolated clinically. Furthermore, since macrolide resistant pneumococci have been highly frequently isolated from community acquired pneumonia patients, it has been considered important that they are effective against the resistant pneumococci.

As a result of various researches in recent years, Agouridas et al. found HMR3647 (telithromycin, Patent document 3) in 1995, and successively Or et al. found ABT-773 (cethromycin, Patent document 4) in 1998 as macrolides that are effective against both erythromycin resistant pneumococci and erythromycin resistant streptococci. Then, 2-fluoroketolide (Patent document 5) of which efficacy was further enhanced was reported.

However, most of the macrolide compounds having a methyl group substituted with a substituent having nitrogen atom at the 4"-position of the cladinose are azalide type compounds structurally characterized by having nitrogen atom in the lactone ring (Patent document 6).

Furthermore, as macrolides effective against both erythromycin resistant pneumococci and erythromycin resistant streptococci, the applicant of this application also reported macrolide compounds having a methyl group substituted with a substituent having nitrogen atom at the 4"-position of the cladinose (Patent documents 7, 8, and 9). Among those compounds, the compound described in Patent documents 7 and 8, Example 15 is an especially preferred compound.

### Prior Art Documents

### Patent Documents

Patent document 1: U.S. Patent No. 4,474,768
Patent document 2: U.S. Patent No. 4,517,359
Patent document 3: EP680967
Patent document 4: WO98/09978
Patent document 5: WO02/32919
Patent document 6: WO98/56801
Patent document 7: WO2012/115256
Patent document 8: JP2014-505723A (Kohyo)
Patent document 9: JP2014-058509A (Kokai)

### Disclosure of the Invention

### Object to be Achieved by the Invention

An object of the present invention is to provide a compound effective against not only conventional erythromycin susceptible bacteria, but also erythromycin resistant bacteria (for example, resistant pneumococci, resistant streptococci, and mycoplasmas).

Therefore, the inventors of the present invention conducted various researches on novel macrolide compounds, and as a result, found that the compound described below had superior antibacterial activity and accomplished the present invention.

The present invention thus provides:
(1) A compound represented by the following formula [1]: 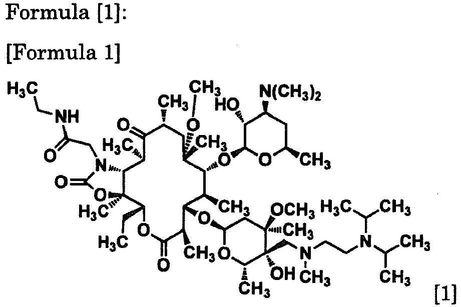 or a pharmaceutically acceptable salt thereof, or a hydrate or a solvate thereof, as a compound that achieves the aforementioned object.

The present invention also provides an agent for prophylactic and/or therapeutic treatment of an infectious disease, which contains the compound as an active ingredient.

### Effect of the Invention

The compound of the present invention, salts thereof, hydrates thereof, and solvates thereof have an antibacterial activity against a wide variety of microorganisms, preferably aerobic or anaerobic bacteria such as Gram-positive or Gram-negative bacteria, mycoplasmas, chlamydiae, and the like, and they are characterized in, in particular, that they have superior antibacterial activity also against erythromycin resistant bacteria (for example, resistant pneumococci, resistant streptococci and mycoplasmas), and the like, against which sufficient antibacterial activity cannot be obtained with conventional macrolide antibiotics.

### Brief Description of the Drawings

- Fig. 1: is a graph showing the results of Test Example 3.
- Fig. 2: is a graph showing the results of Test Example 4.
- Fig. 3: is a graph showing the results of Test Example 5.

### Best Mode for Carrying out the Invention

In the present invention, the "pharmaceutically acceptable salt" may be an acid addition salt or a base addition salt. Examples of the acid addition salt include, for example, salts with an acid such as acetic acid, propionic acid, butyric acid, formic acid, trifluoroacetic acid, maleic acid, tartaric acid, citric acid, stearic acid, succinic acid, ethylsuccinic acid, lactobionic acid, gluconic acid, glucoheptonic acid, benzoic acid, methanesulfonic acid, ethanesulfonic acid, 2-hydroxyethanesulfonic acid, benzenesulfonic acid, paratoluenesulfonic acid, laurylsulfuric acid, malic acid, aspartic acid, glutamic acid, adipic acid, cysteine, N-acetylcysteine, hydrochloric acid, hydrobromic acid, phosphoric acid, sulfuric acid, hydroiodic acid, nicotinic acid, oxalic acid, picric acid, thiocyanic acid, undecanoic acid, acrylic acid polymer, and carboxyvinyl polymer, and examples of the base addition salt include salts with an inorganic base such as sodium salts, potassium salts and calcium salts, salts with an organic amine such as morpholine and piperidine, and salts with an amino acid, but the salt is not limited to these.

In the present invention, the "antibacterial agent" means a substance having an ability to act against bacteria such as Gram-positive or Gram-negative bacteria, and mycoplasmas, and thereby suppress growth thereof or kill them. The term also means a substance that suppresses proliferation of bacteria, or kills a part of bacteria to reduce the number thereof. Examples of the Gram-positive bacteria include, for example, those of the genera *Staphylococcus* (*Staphylococcus aureus, Staphylococcus epidermidis,* and the like), *Streptococcus* (*Streptococcus pyogenes, Streptococcus* group B, *Streptococcus pneumoniae,* and the like), and *Enterococcus* (*Enterococcus faecalis, Enterococcus faecium,* and the like). Examples of the Gram-negative bacteria include, for example, those of the genera *Pseudomonas* (*Pseudomonas aeruginosa* and the like), *Escherichia* (*Escherichia coli* and the like), *Klebsiella* (*Klebsiella pneumoniae, Klebsiella oxytoca,* and the like), *Haemophilus* (*Hemophilus influenzae, Hemophilus parainfluenzae,* and the like), *Bordetella* (*Bordetella pertussis, Bordetella bronchiseptica,* and the like), *Serratia* (*Serratia marcescens* and the like), *Proteus* (*Proteus mirabilis* and the like), *Enterobacter* (*Enterobacter cloaca* and the like), *Campylobacter* (*Campylobacter jejuni* and the like), *Citrobacter, Vibrio* (*Vibrio parahaemolyticus, Vibrio cholerae,* and the like), *Morganella* (*Morganella morganii* and the like), *Salmonella* (*Salmonella typhi, Salmonella paratyphi,* and the like), *Shigella* (*Shigella dysenteriae* and the like), *Acinetobacter* (*Acinetobacter baumannii, Acinetobacter calcoaceticus,* and the like), *Legionella* (*Legionella pneumophila,* and the like), *Bacteroides* (*Bacteroides fragilis* and the like), *Neisseria* (*Neisseria gonorrhoeae, Neisseria meningitidis,* and the like), *Moraxella* (*Moraxella catarrhalis* and the like), *Chlamydia (Chlamydia trachomatis, Chlamydia psittaci* etc,), and *Helicobacter* (*Helicobacter pylori* and the like). Examples of mycoplasma include *M. gallisepticum, M. genitalium, M. hominis, M. hyopneumoniae, M. laboratorium, M, mycoides, M. ovipneumoniae,* and *M. pneumonia.*

The compound of the present invention has a characteristic of exhibiting a superior antibacterial activity also against, especially, erythromycin resistant bacteria (for example, resistant pneumococci, resistant streptococci and mycoplasmas), and the like, against which sufficient antibacterial activity cannot be obtained with conventional macrolide antibiotics.

There may exist optical isomers of the compound represented by the aforementioned formula [1], and such optical isomers and a mixture of such optical isomers are encompassed within the scope of the compound represented by the formula [1]. The compound represented by the formula [1], pharmaceutically acceptable salts thereof, and various hydrates and solvates thereof also fall within the scope of the present invention.

The term "solvent" of the "solvate" referred to in the present invention means, unless specifically indicated, for example, a polar solvent (e.g., an alcohol type solvent such as methanol, ethanol, 1-propanol, 2-propanol, and butanol, ethyl acetate, and the like), an inert solvent (e.g., a halogenated hydrocarbon type solvent such as chloroform and methylene chloride, an ether type solvent such as diethyl ether, tetrahydrofuran and dioxane, an amide type solvent such as dimethylformamide and dimethylacetamide, an aprotic solvent such as dimethyl sulfoxide and acetonitrile, an aromatic hydrocarbon type solvent such as toluene, a hydrocarbon such as cyclohexane, and the like), 2-butanone, hexane, isopropyl ether, acetone, dichloromethane, or the like, or a mixed solvent of any of the solvents exemplified above, but the solvent is not limited to these examples.

The compounds of the present invention represented by the aforementioned formula [1], salts thereof, hydrates or solvates thereof have superior safety. The safety can be evaluated by various tests, for example, cytotoxic test, hERG test, cytochrome P-450 (CYP) activity inhibition test, and the like.

The compounds of the present invention represented by the aforementioned formula [1], salts thereof, hydrates or solvates thereof have superior metabolic stability. The metabolic stability can be evaluated by various tests, for example, human hepatic microsome metabolic stability test, and the like.

The compound of the present invention can be combined with one or two or more kinds of pharmaceutically acceptable carriers, excipients, or diluents to form a pharmaceutical preparation. Examples of the aforementioned carriers, excipients, and diluents include water, lactose, dextrose, fructose, glucose, sucrose, sorbitol, mannitol, polyethylene glycol, propylene glycol, starch, gum, gelatin, alginate, calcium silicate, calcium phosphate, aqueous syrup, cellulose, methylcellulose, hydroxypropylcellulose, polyvinylpyrrolidone, alkyl para-hydroxybenzosorbate, talc, magnesium stearate, stearic acid, myristic acid, glycerin, various oils such as sesame oil, olive oil, and soybean oil, and the like. The above carriers, excipients or diluents may optionally be blended with commonly used additives such as extenders, binders, disintegrating agents, pH adjustors, solubilizers and the like, and then formulated by using conventional pharmaceutical techniques into oral or parenteral dosage forms including tablets, pills, capsules, granules, powders, solutions, emulsions, suspensions, ointments, injections, skin plasters, and the like.

The compound of the present invention may be administered parenterally or orally to adult patients at a dose of 1 to 10000 mg, preferably 5 to 1000 mg, per administration, once or several times a day. This dose may be appropriately increased or decreased depending on the type of disease to be treated, age, body weight and symptom of patient, and the like. The compound of the present invention may also be used in combination with other medicaments.

### Examples

The present invention will be more specifically explained with reference to reference examples, examples and test example. The synthetic method of the compound of the present invention is not limited to the following method, and the compound can be synthesized with any modifications well known to those skilled in the art, such as changing the order of synthesis steps, and performing protection and deprotection of functional groups.

The instrumental data mentioned in the following reference examples and examples were measured with the following measurement apparatuses. NMR spectrum: JNM-ECA600 (600 MHz, JEOL), JNM-ECA500 (500 MHz, JEOL) MS spectrum: LCMS-2010EV (Shimadzu) or Platform LC (Micromass)

In the following reference examples and examples, the high performance liquid chromatography mass spectra (LCMS) were measured under the following conditions.
Measurement apparatus: Agilent 2900 and Agilent 6150
Column: Waters Acquity CSH C18, 1.7 µm, ϕ 2.1 x 50 mm
Solvent: Solution A, water containing 0.1% formic acid; Solution B, acetonitrile containing 0.1% formic acid

### (Condition 1)

Gradient: 0 minute (Solution A/Solution B = 80/20), 1.2 to 1.4 minutes (Solution A/Solution B = 1/99)
Flow rate: 0.8 mL/minute
Detection method: UV, ELSD

### (Condition 2)

Gradient: 0 minute (Solution A/Solution B = 95/5), 1.20 minutes (Solution A/Solution B = 50/50), 1.0 mL/minute, 1.38 minutes (Solution A/Solution B = 3/97)
Flow rate: 0.8 mL/minute
Detection method: UV, ELSD
Ionizing method: ESI

Meanings of the abbreviations used in the reference examples and examples are as follows.
ESI: Electro-spray ionization method
MS: Mass spectrum
CDCl₃: Deuterated chloroform
NMR: Nuclear magnetic resonance
s: Singlet
brs: Broad singlet (singlet having a large width)
d: Doublet
m: Multiplet
t: Triplet
q: Quartet

### Reference Example 1: Synthesis of N,N-diisopropyl-N-methylethane-1,2-diamine

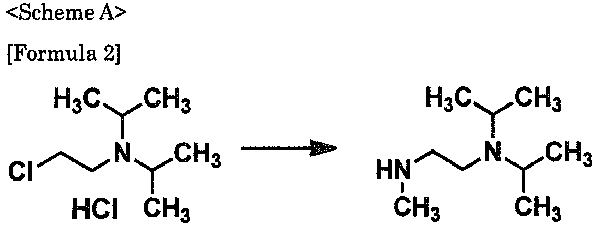

To a 8.9 mol/L solution of methylamine in methanol (135 mL), a solution of diisopropylaminoethyl chloride hydrochloride (24.0 g) in methanol (72 mL) was added dropwise under ice cooling, and the resulting mixture was stirred at room temperature for 20 minutes. The reaction mixture was concentrated under reduced pressure, the resulting residue was dissolved in chloroform, and 2 mol/L aqueous sodium hydroxide was added to the solution under ice cooling. The reaction mixture was extracted twice with chloroform, the organic layer was concentrated under reduced pressure, and the resulting residue was purified by amino silica gel column chromatography (hexane:chloroform = 5:1 to chloroform alone) to obtain the title compound (19.4 g). MS (ESI) m/z = 159 [M+H]⁺
¹H-NMR (400 MHz, CDCl₃) δ (ppm): 0.99 (d, J=1.71Hz, 6H), 1.00 (d, J=1.71Hz, 6H), 2.43 (s, 3H), 2.54 -2.57 (m, 4H), 2.96 -3.03 (m, 2H)

### Reference Example 2: Synthesis of 2-amino-N-ethylacetamide

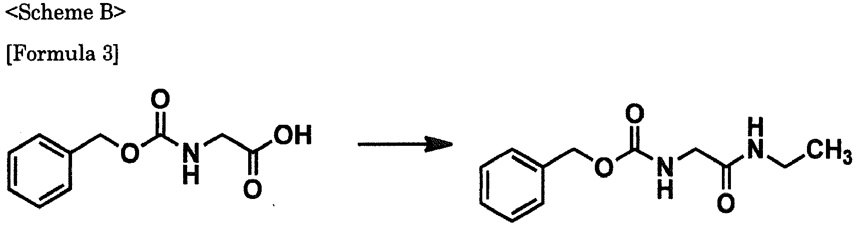

(1) To a solution of N-(benzyloxycarbonyl)glycine (209 g) in chloroform (1.0 L), 70% aqueous ethylamine (108 mL) was added, 1-ethyl-3-(3-dimethylaminopropyl)-carbodiimide hydrochloride (249 g) was added to the mixture under ice cooling, and the resulting mixture was stirred overnight at room temperature. Saturated aqueous sodium hydrogencarbonate was added to the reaction mixture, and the resulting mixture was extracted with chloroform. The organic layer was concentrated under reduced pressure, and then the resulting residue was suspended in ethyl acetate (400 mL). Hexane (200 mL) was added to the suspension, the resulting mixture was stirred, and the deposited solid was collected by filtration to obtain an amide compound (150 g). 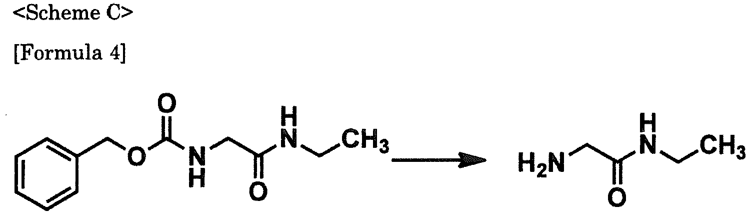
(2) To a solution of the amide compound (150 g) obtained in Reference Example 2, (1) mentioned above in methanol (630 mL), 10% palladium/carbon (15 g) was added, and the mixture was stirred at room temperature for 6 days under a hydrogen atmosphere. The reaction mixture was filtered, and then the filtrate was concentrated under reduced pressure to obtain the title compound (64.4 g).
   MS (ESI) m/z = 103 [M+H]⁺
   ¹H-NMR (400 MHz, CDCl₃) δ (ppm): 1.17 (t, J=7.2Hz, 3H), 1.38 (brs, 2H), 3.29-3.37 (m, 4H), 7.20 (brs, 1H)

### Reference Example 3: Preparation of the compound represented by the formula [2]:

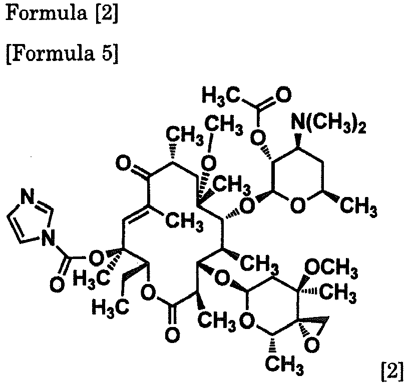

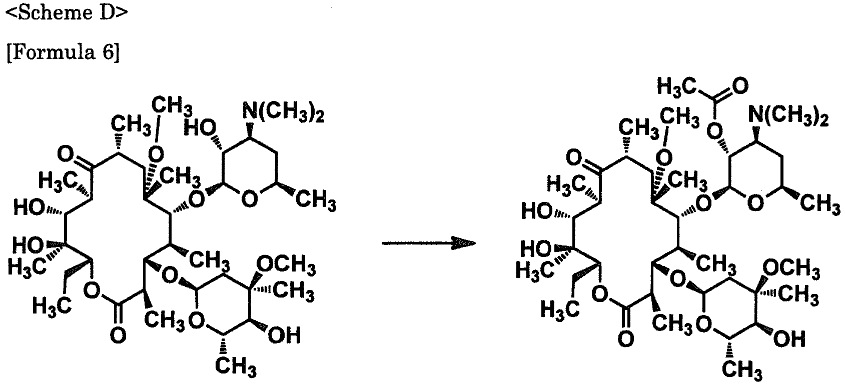

(1) Clarithromycin (200 g) was dissolved in acetone (1.5 L), acetic anhydride (30.3 ml) was added dropwise to the solution, and the resulting mixture was stirred overnight at room temperature. The reaction mixture was concentrated under reduced pressure, ethyl acetate, hexane and aqueous sodium hydroxide were added to the resulting residue, and then saturated aqueous sodium hydrogencarbonate was added to the mixture to adjust the mixture to pH 9. The deposited solid was collected by filtration with a glass filter, washed with distilled water, and then dried under reduced pressure to obtain an acetyl compound (202 g).
   MS (ESI) m/z = 790.6 [M+H]⁺ 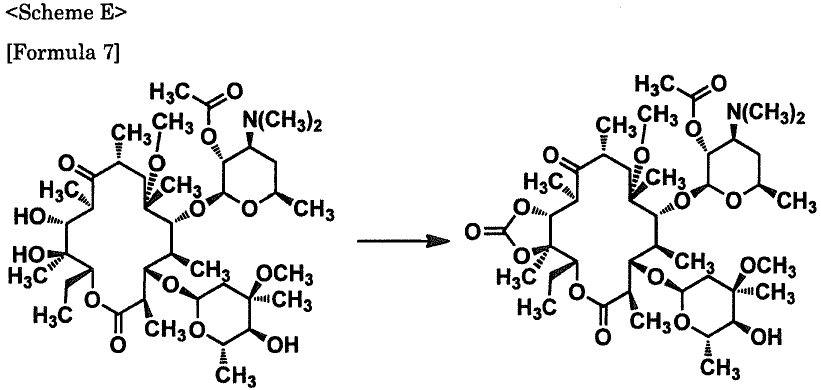
(2) The acetyl compound obtained in Reference Example 3, (1) mentioned above (202 g) was dissolved in chloroform (1.8 L), pyridine (210 ml) was added to the solution, then the resulting mixture was cooled on ice, and a solution of triphosgene (77.4 g) in chloroform (0.8 L) was added dropwise to the mixture over 40 minutes. The reaction mixture was warmed to room temperature, and then stirred for 3 hours. Pyridine (158 ml) was added to the reaction mixture, a solution of triphosgene (57.9 g) in chloroform was added dropwise to the resulting mixture under ice cooling, and the resulting mixture was stirred at room temperature for 15 minutes. Distilled water and saturated aqueous sodium hydrogencarbonate were added to the reaction mixture, the resulting mixture was extracted with chloroform, and the organic layer was dried over anhydrous magnesium sulfate, and filtered. The filtrate was concentrated under reduced pressure, and a mixed solvent of ethyl acetate and hexane (1:1) was added to the resulting residue. The resulting mixture was stirred, hexane was further added to the mixture, and the resulting mixture was stirred overnight at room temperature. The deposited solid was collected by filtration, washed with a mixed solvent of ethyl acetate and hexane (1:2), and then dried under reduced pressure to obtain a carbonate compound (220 g).
   MS (ESI) m/z = 816.5 [M+H]⁺ 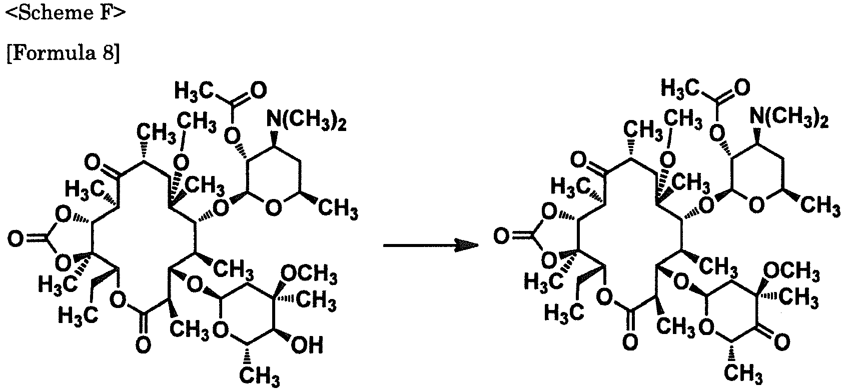
(3) N-Chlorosuccinimide (99.7 g) was dissolved in chloroform (1 L), and the solution was cooled to -25°C. A solution of dimethyl sulfide (210 ml) in chloroform (0.2 L) was added dropwise to the reaction mixture over 20 minutes, and the resulting mixture was stirred for 15 minutes. Then, a solution of the carbonate compound obtained in (2) mentioned above in chloroform (1 L) was added dropwise to the reaction mixture over 30 minutes, and the resulting mixture was stirred for 15 minutes. A solution of triethylamine (136 ml) in chloroform (0.2 L) was added to the reaction mixture, and the resulting mixture was stirred for 30 minutes. Saturated aqueous sodium hydrogencarbonate was added to the reaction mixture, the resulting mixture was warmed to room temperature, chloroform was added to the mixture, and the layers were separated. The organic layer was dried over anhydrous magnesium sulfate, and filtered, and then the filtrate was concentrated under reduced pressure. A mixed solvent of ethyl acetate and hexane (1:5) was added to the resulting residue, and the resulting mixture was stirred overnight at room temperature. The deposited solid was collected by filtration, and washed with a mixed solvent of ethyl acetate and hexane (1:2) to obtain a ketone compound (109 g). The filtrate was concentrated under reduced pressure, and the resulting residue was purified by silica gel column chromatography (ethyl acetate:hexane = 1:1 to acetone:hexane:triethylamine = 10:10:0.2), and then crystallized in the same manner as that described above to obtain a ketone compound (59.5 g).
   MS (ESI) m/z = 814.5 [M+H]⁺ 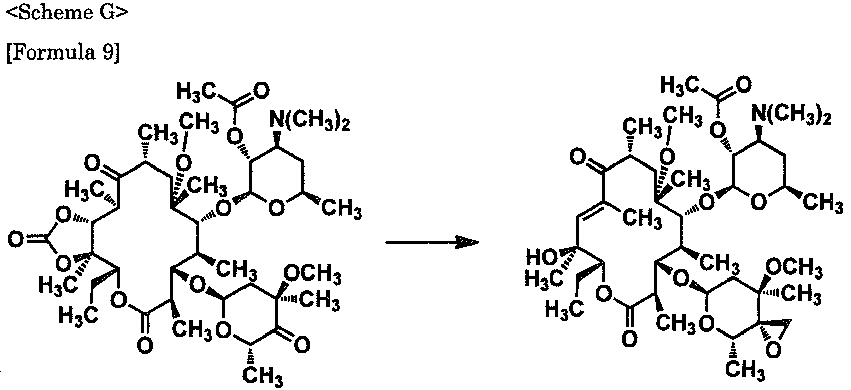
(4) Trimethylsulfoxonium iodide (210 g) was dissolved in a mixed solvent of dimethyl sulfoxide and tetrahydrofuran (5:1, 1.2 L), 70% sodium hydride (32.6 g) was added portionwise to the solution, and the resulting mixture was stirred at room temperature for 1.5 hours. A solution of the ketone compound obtained in (3) mentioned above (155 g) in tetrahydrofuran (0.8 L) was added dropwise to the reaction mixture under ice cooling, and the resulting mixture was stirred at room temperature for 30 minutes. The reaction mixture was cooled on ice, distilled water and ethyl acetate were added to the reaction mixture, and the layers were separated. The resulting organic layer was washed with distilled water. The aqueous layer was extracted with ethyl acetate, and the organic layer was washed with distilled water. The organic layers were combined, dried over anhydrous magnesium sulfate, and filtered. The filtrate was concentrated under reduced pressure to obtain an epoxy compound (146 g).
   MS (ESI) m/z = 784.5 [M+H]⁺
   ¹H-NMR (600 MHz, CDCl₃) δ (ppm): 0.90 (t, J=7.57Hz, 3H), 0.97 (d, J=7.34Hz, 3H), 1.04 (d, J=6.88Hz, 3H), 1.07 (s, 3H), 1.14 (d, J=6.88Hz, 3H), 1.18 (d, J=5.96Hz, 3H), 1.21-1.36 (m, 7H), 1.42 (s, 3H), 1.47-1.55 (m, 1H), 1.67-1.73 (m, 1H), 1.83-1.98 (m, 5H), 2.02 (d, J=1.83Hz, 6H), 2.18-2.29 (m, 1H), 2.25 (s, 6H), 2.58-2.69 (m, 1H), 2.63 (d, J=4.13Hz, 1H), 2.80-2.89 (m, 1H), 2.94 (d, J=4.13Hz, 1H), 3.12-3.26 (m, 1H), 3.17 (s, 3H), 3.34 (s, 3H), 3.43-3.51 (m, 1H), 3.66 (d, J=6.42Hz, 1H), 3.94 (br. s., 1H), 4.57 (d, J=7.34Hz, 1H), 4.73 (dd, J=10.55, 7.34Hz, 1H), 4.80 (q, J=6.42Hz, 1H), 4.98-5.06 (m, 2H), 6.50 (s, 1H) 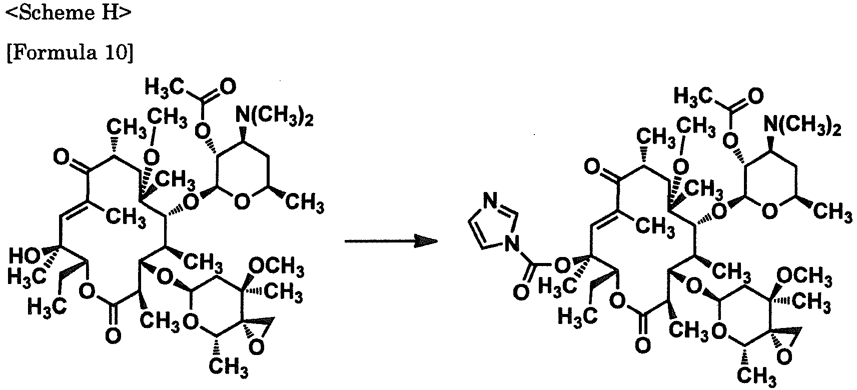
(5) The epoxy compound obtained in Reference Example 3, (4) mentioned above (138 g) was dissolved in a mixed solvent of tetrahydrofuran and dimethylformamide (1:1, 1.4 L), and 1,1'-carbonyldiimidazole (85.6 g) was added to the solution. 70% Sodium hydride (18.1 g) was added to the mixture over 40 minutes under ice cooling, and the resulting mixture was stirred at room temperature for 0.5 hour. The reaction mixture was cooled on ice, and distilled water was added to the reaction mixture. The resulting mixture was extracted with ethyl acetate, and the organic layer was washed twice with distilled water. The aqueous layer was extracted with ethyl acetate, and the organic layer was washed twice with distilled water. The organic layers were combined, dried over anhydrous magnesium sulfate, and filtered. The filtrate was concentrated under reduced pressure, and the resulting residue was purified by silica gel column chromatography (hexane to hexane:ethyl acetate = 1:1 to acetone:hexane:triethylamine = 10:10:0.2). Ethyl acetate and hexane (1:1) were added to the resulting purified product, and the resulting mixture was stirred overnight at room temperature. The deposited solid was collected by filtration, and washed with a mixed solvent of ethyl acetate and hexane (1:4) to obtain the compound represented by the formula [2] (87.1 g).
   MS (ESI) m/z = 878.6 [M+H]⁺
   ¹H-NMR (600 MHz, CDCl₃) δ (ppm): 0.85-1.41 (m, 25H), 1.64-1.78 (m, 3H), 1.79 (s, 3H), 1.90 (dd, J=14.67, 5.04Hz, 4H), 1.86 (s, 3H), 2.04 (s, 3H), 2.19-2.28 (m, 1H), 2.25 (s, 6H), 2.60-2.68 (m, 1H), 2.65 (d, J=4.13Hz, 1H), 2.86-2.97 (m, 1H), 2.95 (d, J=4.13Hz, 1H), 3.15 (s, 3H), 3.22-3.29 (m, 1H), 3.35 (s, 3H), 3.38-3.47 (m, 1H), 3.66 (d, J=6.42Hz, 1H), 3.79-3.88 (m, 1H), 4.56 (d, J=6.88Hz, 1H), 4.72 (dd, J=10.32, 7.57Hz, 1H), 4.79 (q, J=6.27Hz, 1H), 5.01-5.09 (m, 1H), 5.83 (dd, J=10.55, 2.75Hz, 1H), 6.66 (s, 1H), 7.07 (s, 1H), 7.34-7.38 (m, 1H), 8.08 (s, 1H)

### Reference Example 4: Preparation of the compound represented by the formula [3]

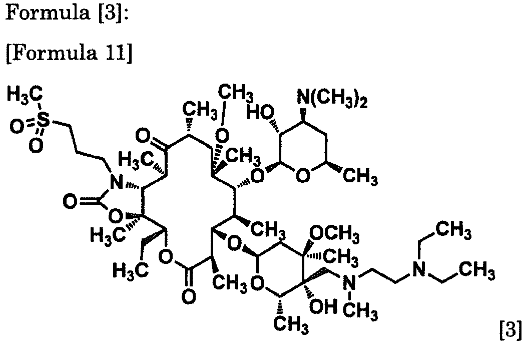

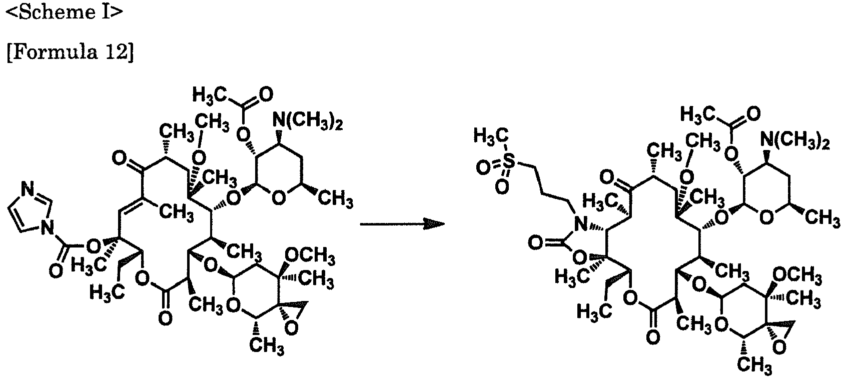

(1) The compound represented by the formula [2] obtained in Reference Example 3 (360 mg) was dissolved in acetonitrile (1.5 ml), 1,8-diazabicyclo[5,4,0]-7-undecene (280 µl) and 3-methanesulfonylpropylamine hydrochloride (273 mg) were added to the solution, and the resulting mixture was stirred at room temperature for 1 day. Ethyl acetate and saturated aqueous ammonium chloride were added to the reaction mixture, and the layers were separated. The organic layer was dried over anhydrous magnesium sulfate, and filtered, the filtrate was concentrated under reduced pressure, and the resulting residue was purified by silica gel column chromatography (chloroform to chloroform:methanol:28% aqueous ammonia = 25:1:0.1 to 15:1:0.1) to obtain a carbamate compound (117 mg). 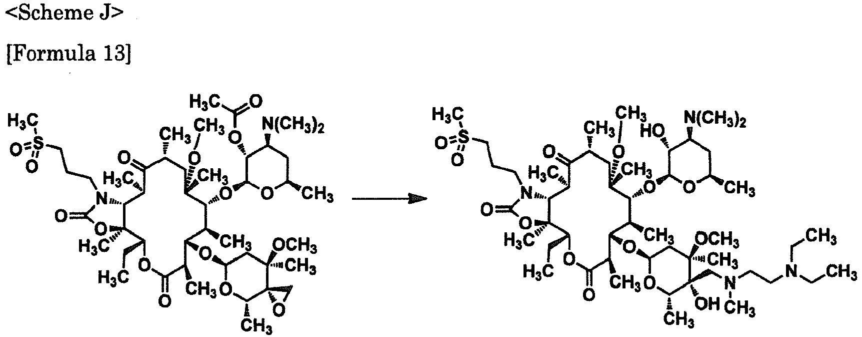
(2) The carbamate compound obtained in Reference Example 4, (1) mentioned above (115 mg) was dissolved in ethanol (1 ml), N,N-diethyl-N-methylethane-1,2-diamine (195 µl) was added to the solution, and the resulting mixture was stirred at 100°C for 1 day in a sealed tube. Ethyl acetate and saturated aqueous ammonium chloride were added to the reaction mixture, and the layers were separated. The organic layer was dried over anhydrous magnesium sulfate, and filtered, the filtrate was concentrated under reduced pressure, and the resulting residue was purified by silica gel column chromatography (chloroform to chloroform:methanol:28% aqueous ammonia = 12:1:0.1) and preparative thin layer chromatography (chloroform:methanol:28% aqueous ammonia = 20:1:0.1) to obtain the compound represented by the formula [3] (62.7 mg).
   The compound represented by the formula [3] is the compound described in Patent documents 7 and 8, Example 15 as a preferred compound.

### Example 1: Preparation of the compound represented by the formula [1]

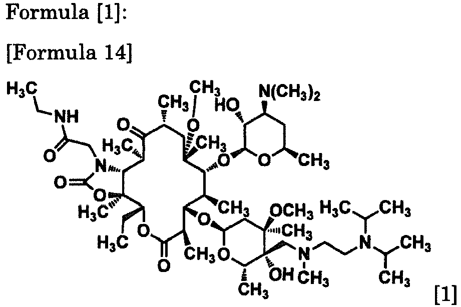

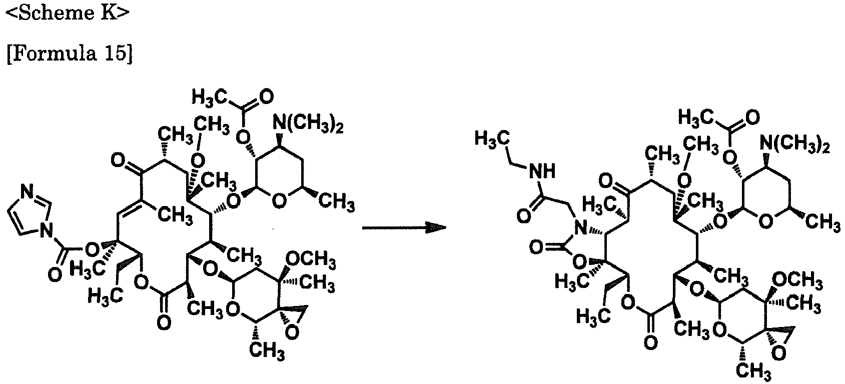

(1) The compound represented by the formula [2] obtained in Reference Example 3 (277 g) was dissolved in acetonitrile (315 mL), the compound obtained in Reference Example 2 (64.4 g) and 1,8-diazabicyclo[5,4,0]-7-undecene (191 mL) were added to the solution, and the resulting mixture was stirred at room temperature for 1.5 hours. Water (500 mL) was added to the reaction mixture, and the resulting mixture was extracted with ethyl acetate (400 mL). The organic layer was washed with saturated brine, dried over magnesium sulfate, filtered, and then concentrated under reduced pressure. The residue was recrystallized from ethyl acetate (300 mL) and hexane (300 mL) to obtain a carbamate compound (83.5 g). The filtrate was concentrated under reduced pressure, and the residue was recrystallized (ethyl acetate (200 mL), hexane (200 mL)) to obtain the carbamate compound (34.4 g). The filtrate was further concentrated under reduced pressure, and the residue was purified by silica gel column chromatography (chloroform:methanol:28% aqueous ammonia = 99:1:0.1 to 85:15:1.5), and then recrystallized from ethyl acetate (100 mL) and hexane (100 mL) to obtain the carbamate compound (16.3 g). The filtrate and a part of the fractions obtained from the aforementioned column were combined, purified by amino silica gel column chromatography (ethyl acetate:hexane = 20:80 to ethyl acetate alone), and then recrystallized from ethyl acetate (200 mL) and hexane (200 mL) to obtain the carbamate compound (55.3 g). The carbamate compound was obtained in a total amount of 189.5 g.
   MS (ESI) m/z = 912.6 [M+H]⁺
   ¹H-NMR (499 MHz, CDCl₃) δ (ppm): 0.85-0.90 (m, 6H), 0.95 (d, J=7.55Hz, 3H), 0.99-1.29 (m, 19H), 1.33 (s, 3H), 1.44 (s, 3H), 1.50-1.65 (m, 3H), 1.68-1.73 (m, 1H), 1.84-2.00 (m, 3H), 2.05 (s, 3H), 2.21 (dd, J=14.75, 3.09Hz, 1H), 2.26 (s, 6H), 2.53-2.60 (m, 1H), 2.62 (d, J=4.12Hz, 1H), 2.63-2.70 (m, 1H), 2.86-2.91 (m, 1H), 2.93 (s, 3H), 2.94 (d, J=4.12Hz, 1H), 3.06 (q, J=6.86Hz, 1H), 3.27-3.36 (m, 2H), 3.34 (s, 3H), 3.41-3.50 (m, 1H), 3.68 (d, J=6.17Hz, 1H), 3.73 (d, J=10.29Hz, 1H), 3.76 (s, 1H), 4.20 (d, J=16.81Hz, 1H), 4.49 (d, J=16.81Hz, 1H), 4.63 (d, J=7.55Hz, 1H), 4.68-4.77 (m, 2H), 5.04 (dd, J=4.63, 3.26Hz, 1H), 5.25 (dd, J=10.63, 2.40Hz, 1H), 6.17 (t, J=5.66Hz, 1H) 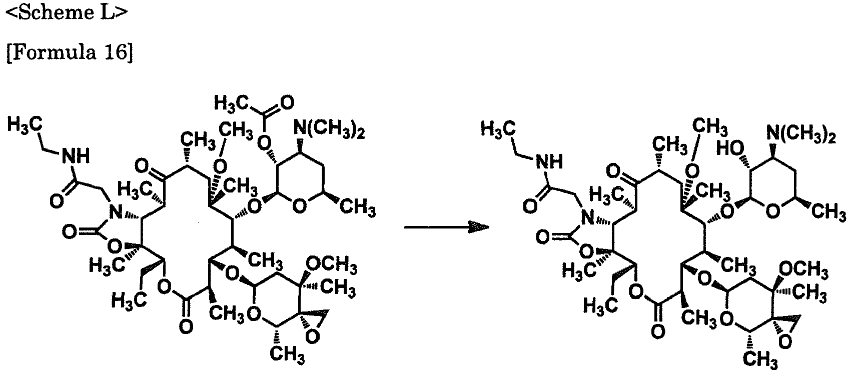
(2) The carbamate compound obtained in Example 1, (1) mentioned above (189 g) was dissolved in methanol (410 mL), and the solution was refluxed by heating for 4 hours, and stirred at room temperature over one whole day and night. The reaction mixture was concentrated under reduced pressure. Ethyl acetate (50 mL) and hexane (300 mL) were added to the residue, the resulting mixture was stirred for 30 minutes, and the deposited solid was collected by filtration to obtain a deacetylated compound (41.2 g). The filtrate was purified by amino silica gel column chromatography (ethyl acetate:hexane = 20:80 to 100:0) and silica gel column chromatography (chloroform:methanol:28% aqueous ammonia = 99:1:0.1 to 85:15:1.5) 3 times. Ethyl acetate (50 mL) and hexane (600 mL) were added to the resulting roughly purified product, the resulting mixture was stirred for 30 minutes, and the deposited solid was collected by filtration to obtain the deacetylated compound (62.8 g). The filtrate was further purified by silica gel column chromatography (chloroform:methanol:28% aqueous ammonia = 99:1:0.1 to 85:15:1.5), and similarly recrystallized from ethyl acetate (20 mL) and hexane (50 mL) to obtain the deacetylated compound (2.99 g). MS (ESI) m/z = 870.6 [M+H]⁺
   ¹H-NMR (499 MHz, CDCl₃) δ (ppm): 0.88 (t, J=7.38Hz, 3H), 1.00-1.08 (m, 9H), 1.09-1.27 (m, 1H), 1.10-1.15 (m, 9H), 1.18 (d, J=6.17Hz, 3H), 1.24 (d, J=7.20Hz, 3H), 1.36 (s, 3H), 1.43 (s, 3H), 1.58 (ddd, J=14.24, 10.46, 7.20Hz, 1H), 1.62-1.78 (m, 3H), 1.88 (dd, J=14.92, 4.97Hz, 1H), 1.91-2.00 (m, 2H), 2.23 (dd, J=14.75, 2.74Hz, 1H), 2.28 (s, 6H), 2.42-2.50 (m, 1H), 2.59 (dd, J=7.03, 4.29Hz, 1H), 2.62 (d, J=4.12Hz, 1H), 2.89-2.96 (m, 1H), 2.93 (d, J=4.12Hz, 1H), 2.95 (s, 3H), 3.08 (q, J=6.86Hz, 1H), 3.18 (dd, J=10.29, 7.20Hz, 1H), 3.27-3.39 (m, 2H), 3.32 (s, 3H), 3.42-3.50 (m, 1H), 3.71 (d, J=6.52Hz, 1H), 3.76 (d, J=9.95Hz, 1H), 3.77 (s, 1H), 4.21 (d, J=16.81Hz, 1H), 4.50 (d, J=10.63Hz, 1H), 4.52 (s, 1H), 4.76 (q, J=6.52Hz, 1H), 5.04 (dd, J=4.80, 2.74Hz, 1H), 5.21 (dd, J=10.63, 2.40Hz, 1H), 6.25 (t, J=5.66Hz, 1H) 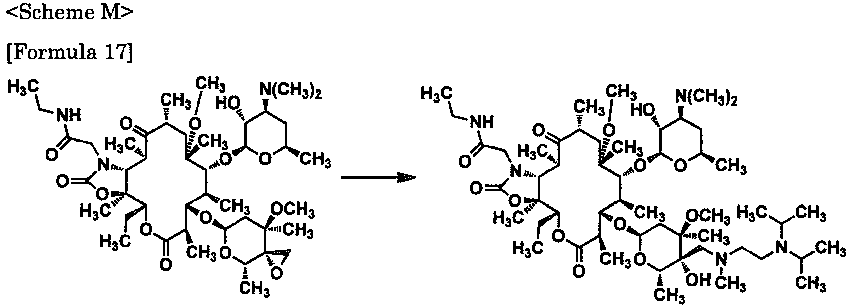
(3) The deacetylated compound obtained in Example 1, (2) mentioned above (104 g) was dissolved in ethanol (120 mL), the compound obtained in Reference Example 1 (56.5 g) was added to the solution, and the resulting mixture was refluxed by heating for 2 hours. The reaction mixture was concentrated under reduced pressure. The residue was dissolved in ethyl acetate, the solution was washed 3 times with saturated aqueous sodium hydrogencarbonate, water was added to the solution, and the layers were separated. The aqueous layer was extracted again with ethyl acetate, and the organic layer was washed with water. The organic layers were combined, washed with saturated brine, dried over magnesium sulfate, filtered, and concentrated under reduced pressure. The residue was recrystallized from ethyl acetate (100 mL) and hexane (600 mL) to obtain the compound represented by the formula [1] (41.4 g). The filtrate was further concentrated under reduced pressure, and the residue was purified by silica gel column chromatography (chloroform:methanol:28% aqueous ammonia = 99:1:0.1 to 85:15:1.5), and recrystallized from ethyl acetate (100 mL) and hexane (500 mL) to obtain the compound represented by the formula [1] (62.1 g). The portions of the compound represented by the formula [1] obtained as described above were combined. The compound represented by the formula [1] was obtained in a total amount of 103.5 g.
   MS (ESI) m/z = 1028.8 [M+H]⁺
   ¹H-NMR (499 MHz, CDCl₃) δ (ppm): 0.87 (t, J=7.20Hz, 3H), 1.00 (m, J=10.60, 6.50Hz, 15H), 1.06-1.26 (m, 22H), 1.38 (s, 3H), 1.42 (s, 3H), 1.52-1.79 (m, 4H), 1.84-2.07 (m, 5H), 2.29 (s, 6H), 2.35 (s, 3H), 2.39-2.55 (m, 5H), 2.57-2.64 (m, 1H), 2.83 (d, J=14.75Hz, 1H), 2.89 (dd, J=9.26, 7.20Hz, 1H), 2.94 (s, 3H), 2.95-3.03 (m, 2H), 3.08 (q, J=7.09Hz, 1H), 3.17 (dd, J=10.12, 7.38Hz, 1H), 3.22-3.32 (m, 1H), 3.28 (s, 3H), 3.34-3.48 (m, 3H), 3.64 (d, J=7.55Hz, 1H), 3.73 (d, J=9.61Hz, 1H), 3.78 (s, 1H), 4.08 (q, J=6.40Hz, 1H), 4.21 (d, J=17.15Hz, 1H), 4.40 (d, J=7.20Hz, 1H), 4.57 (d, J=16.81Hz, 1H), 4.95 (brs, 1H), 4.99 (d, J=4.80Hz, 1H), 5.11 (dd, J=10.63, 2.06Hz, 1H), 6.39 (t, J=5.66Hz, 1H)

The action of the compound of the present invention was confirmed by the following pharmacological tests.

### Test Example 1: In vitro antibacterial activity

*In vitro* antibacterial activities of the compound of the present invention, the compound represented by the formula [1] of Example 1, against various test bacteria were measured according to the microbroth dilution method (CLSI method). The activities of the compound of Reference Example 4 represented by the formula [3] was similarly measured. The used test bacteria are shown in Table 1. The MIC values (minimum inhibitory concentration, µg/ml) for the test bacteria of the bacterium symbols A, B, C, D, E, F, G, H, I, J, K, and L are shown in Table 2.

**[Table 1]**

| Test bacteria | Symbol of Bacteria |
|---|---|
| Haemophilus influenzae ATCC43095 | A |
| Haemophilus influenzae MSC17946 | B |
| Haemophilus influenzae NSC17960 | C |
| Streptococcus pneumoniae ATCC49619 | D |
| Streptococcus pneumoniae MSC07465 | E |
| Streptococcus pneumoniae MSC08607 | F |
| Streptococcus pneumoniae ATCC700904 | G |
| Streptococcus pneumoniae MSC07365 | H |
| Streptococcus pyogenes ATCC12344 | I |
| Streptococcus pyogenes MSC07812 | J |
| Streptococcus pyogenes MSC07811 | K |
| Streptococcus aureus ATCC29213 | L |

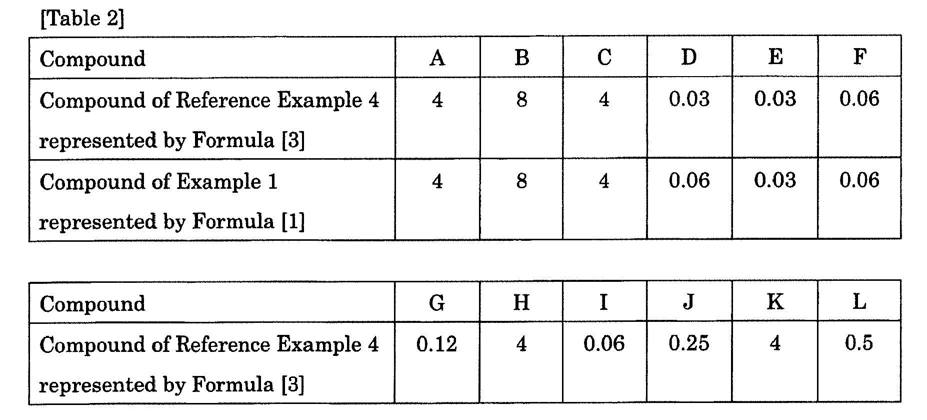

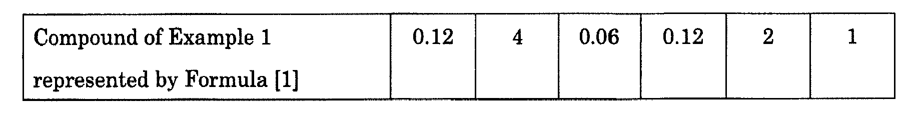

### Test Example 2: Hemophilus influenzae susceptibility test

Drug susceptibility of 39 kinds of clinically isolated strains of *Hemophilus influenzae* was evaluated in the same manner as that of Test Example 1. The results are shown in Table 3.

**[Table 3]**

| Compound | MIC50 | MIC90 |
|---|---|---|
| Compound of Reference Example 4 represented by Formula [3] | 4 | 8 |
| Compound of Example 1 represented by Formula [1] | 4 | 8 |

### Test Example 3: Test evaluating therapeutic effectiveness in Haemophilus influenzae-infected animals

For evaluating the pharmacological effectiveness, the following method was used.

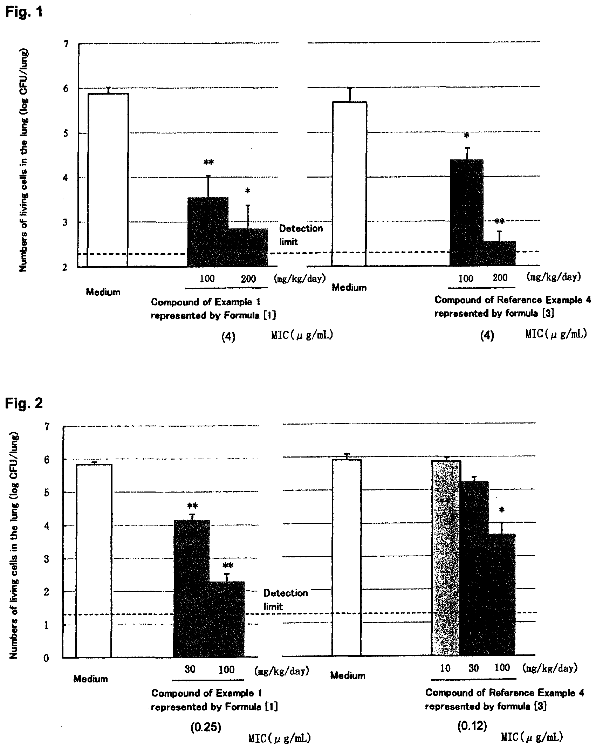

As a bacterium, the *Haemophilus influenzae* ATCC 43095 strain (bacterium symbol A) was used. Cells of the bacterium cultured overnight on a chocolate agar medium were collected, suspended in a *Hemophilus* susceptibility test medium or the brain heart infusion medium supplemented with Fildes Enrichment, and then cultured overnight. The culture was diluted with the *Hemophilus* susceptibility test medium or the brain heart infusion medium supplemented with Fildes Enrichment to prepare a bacterial suspension for inoculation. The bacterial suspension for inoculation (0.05 mL each) was intratracheobronchially administered to mice (ICR, male, 4 weeks old) for infection. The inoculated bacterial amount was 2.25 x 10⁶ CFU/mouse, or 9.00 x 10⁵ CFU/mouse. From the next day of the inoculation, the compound represented by the formula [1] of Example 1 (100 and 200 mg/kg) or a medium (mixture of equivalent volumes of a 0.1 mol/L lactobionic acid solution and a 0.5 w/v% sodium hydrogencarbonate solution) was orally administered to the mice once a day for 2 days. The numbers of living cells in the lung observed 3 days after the inoculation are shown in Fig. 1 (each group consisted of 6 mice, average ± standard error).

The notes for Fig. 1 are as follows. As for significant difference relative to the medium administration group, significance was determined by the Steel test, and the symbols * and ** mean p < 0.05 and p < 0.01, respectively. The MIC value of the compound of Example 1 represented by the formula [1] was 4 µg/mL, and the MIC value of the compound of Reference Example 4 represented by the formula [3] was 4 µg/mL.

In the following descriptions, the numbers of living cells in the lung as the test results may be represented in terms of common logarithm of number of live cells in the lung (CFU/lung) (common logarithm is henceforth indicated as log).

The number of live cells in the lung of the medium administration group was 5.88 ± 0.14 [log (CFU/lung)]. The numbers of living cells in the lung of the groups administered with 100 and 200 mg/kg of the compound of Example 1 represented by the formula [1] were 3.54 ± 0.49 [log (CFU/lung)] and 2.83 ± 0.53 [log (CFU/lung)], respectively, and thus the number significantly decreased compared with the medium administration group. Similarly, the compound of Reference Example 4 represented by the formula [3] (100 and 200 mg/kg) or the medium (mixture of equivalent volumes of a 0.1 mol/L lactobionic acid solution and a 0.5 w/v% sodium hydrogencarbonate solution) was orally administered. The results represented in terms of common logarithm (common logarithm is henceforth indicated as log) of number of living cells in the lung (CFU/lung) observed 3 days after the inoculation were 5.67 ± 0.32 [log (CFU/lung)] for the medium administration group, 4.37 ± 0.27 [log (CFU/lung)] and 2.53 ± 0.23 [log (CFU/lung)] for the groups administered with 100 and 200 mg/kg of the compound of Reference Example 4 represented by the formula [3], respectively, and thus the number of living cells significantly decreased in the lung of the compound administration groups compared with the medium administration group. As seen from the results described above, the compound of Example 1 represented by the formula [1] gave the therapeutic effectiveness against the strain comparable to that of the compound of Reference Example 4 represented by the formula [3].

### Test Example 4: Therapeutic effect test in erythromycin resistant pneumococcus (harboring erm(B) gene)-infected animals

For evaluating the pharmacological effectiveness, the following method was used.

As a bacterium, the *Streptococcus pneumoniae* 1101 strain (clinically isolated strain) was used. A frozen stock of the strain used was added to the Todd Hewitt broth supplemented with 30 vol % inactivated horse serum, and the bacterium was cultured until the turbidity (OD600) became about 0.3. The culture medium was diluted with the Todd Hewitt broth supplemented with 30 vol % inactivated horse serum to prepare a bacterial suspension for inoculation. The bacterial suspension for inoculation (0.05 mL each) was intranasally administered to mice (CBA/JN, male, 5 weeks old) for infection. The inoculated bacterial amount was 7.50 x 10⁴ CFU/mouse, or 1.65 x 10⁵ CFU/mouse. From the next day of the inoculation, the compound of Example 1 represented by the formula [1] (30 and 100 mg/kg) or a medium (mixture of equivalent volumes of a 0.1 mol/L lactobionic acid solution and a 0.5 w/v% sodium hydrogencarbonate solution) was orally administered to the mice once a day for 2 days. The numbers of live cells in the lung observed 3 days after the inoculation are shown in Fig. 2 (each group consisted of 5 or 6 mice, average ± standard error).

The notes for Fig. 2 are as follows. As for significant difference relative to the medium administration group, significance was determined by the Steel test, and the symbols * and ** mean p < 0.05 and p < 0.01, respectively. The MIC value of the compound of Example 1 represented by the formula [1] was 0.25 µg/mL, and the MIC value of the compound of Reference Example 4 represented by the formula [3] was 0.12 µg/mL.

The number of living cells in the lung of the medium administration group was 5.83 ± 0.08 [log (CFU/lung)]. The numbers of living cells in the lung of the groups administered with 30 and 100 mg/kg of the compound of Example 1 represented by the formula [1] were 4.14 ± 0.19 [log (CFU/lung)] and 2.28 ± 0.24 [log (CFU/lung)], respectively, and thus the number significantly decreased compared with the medium administration group. Similarly, the compound of Reference Example 4 represented by the formula [3] (10, 30 and 100 mg/kg) or the medium (mixture of equivalent volumes of a 0.1 mol/L lactobionic acid solution and a 0.5 w/v% sodium hydrogencarbonate solution) was orally administered. As a result, the numbers of living cells in the lung (CFU/lung) observed 3 days after the inoculation were 5.91 ± 0.18 [log (CFU/lung)] for the medium administration group, 5.86 ± 0.12 [log (CFU/lung)], 5.22 ± 0.16 [log (CFU/lung)], and 3.65 ± 0.36 [log (CFU/lung)] for the groups administered with 10, 30 and 100 mg/kg of the compound of Reference Example 4 represented by the formula [3], respectively. Thus, the number of living cells in the lung significantly decreased in the group administered with 100 mg/kg of the compound of Reference Example 4 represented by the formula [3] compared with the medium administration group. As seen from the results described above, the compound of Example 1 represented by the formula [1] showed the therapeutic effectiveness superior to that of the compound of Reference Example 4 represented by the formula [3].

### Test Example 5: Therapeutic effect test in erythromycin resistant pneumococcus (harboring mef(A) gene)-infected animals

For evaluating the pharmacological effect, the following method was used.

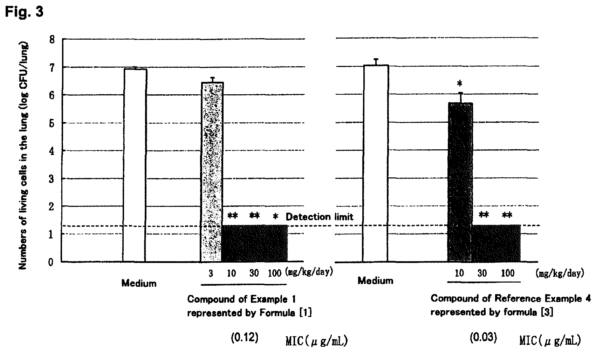

As a bacterium, the *Streptococcus pneumoniae* 1028 strain (clinically isolated strain) was used. A frozen stock of the strain used was added to the Todd Hewitt broth supplemented with 30 vol % inactivated horse serum, and the bacterium was cultured until the turbidity (OD600) became about 0.3. The culture was diluted with the Todd Hewitt broth supplemented with 30 vol % inactivated horse serum to prepare a bacterial suspension for inoculation. The bacterial suspension for inoculation (0.05 mL each) was intranasally administered to mice (CBA/JN, male, 5 weeks old) for infection. The inoculated bacterial amount was 3.45 x 10⁴ CFU/mouse, or 3.90 x 10⁴ CFU/mouse. From the next day of the inoculation, the compound of Example 1 represented by the formula [1] (3, 10, 30 and 100 mg/kg) or a medium (mixture of equivalent volumes of a 0.1 mol/L lactobionic acid solution and a 0.5 w/v% sodium hydrogencarbonate solution) was orally administered to the mice once a day for 2 days. The numbers of living cells in the lung observed 3 days after the inoculation are shown in Fig. 3 (each group consisted of 5 or 6 mice, average ± standard error).

The notes for Fig. 3 are as follows. As for significant difference relative to the medium administration group, significance was determined by the Steel test, and the symbols * and ** mean p < 0.05 and p < 0.01, respectively. The MIC value of the compound of Example 1 represented by the formula [1] was 0.12 µg/mL, and the MIC value of the compound of Reference Example 4 represented by the formula [3] was 0.03 µg/mL.

The number of living cells in the lung of the medium administration group was 6.94 ± 0.07 [log (CFU/lung)]. The numbers of living cells in the lung of the groups administered with 3, 10, 30 and 100 mg/kg of the compound of Example 1 represented by the formula [1] were 6.45 ± 0.18 [log (CFU/lung)], 1.30 ± 0.00 [log (CFU/lung)], 1.30 ± 0.00 [log (CFU/lung)], and 1.30 ± 0.00 [log (CFU/lung)], respectively. Namely, the number of living cells in the lung was lower than detection limit in the groups administered with 10, 30 and 100 mg/kg of the compound of Example 1 represented by the formula [1], and thus the number significantly decreased compared with the medium administration group. Similarly, the compound of Reference Example 4 represented by the formula [3] (10, 30 and 100 mg/kg) or the medium (mixture of equivalent volumes of a 0.1 mol/L lactobionic acid solution and a 0.5 w/v% sodium hydrogencarbonate solution) was orally administered. As a result, the number of living cells in the lung observed 3 days after the inoculation was 7.03 ± 0.22 [log (CFU/lung)] for the medium administration group, and 5.67 ± 0.38 [log (CFU/lung)], 1.30 ± 0.00 [log (CFU/lung)], and 1.30 ± 0.00 [log (CFU/lung)] for the groups administered with 10, 30 and 100 mg/kg of the compound of Reference Example 4 represented by the formula [3], respectively, and thus the number of living cells in the lung significantly decreased in the groups administered with 10, 30 and 100 mg/kg of the compound of Reference Example 4 represented by the formula [3] compared with the medium administration group. However, the groups where the number was lower than the detection limit in all the mice were only the groups administered with 30 and 100 mg/kg of the compound of Reference Example 4 represented by the formula [3]. As seen from the results described above, the compound of Example 1 represented by the formula [1] showed the therapeutic effectiveness superior to that of the compound of Reference Example 4 represented by the formula [3].

### Industrial Applicability

The compound of the present invention, or a pharmaceutically acceptable salt thereof exhibits potent antibacterial activity against Gram-positive bacteria, Gram-negative bacteria, and mycoplasmas, and in particular, it also exhibits superior antibacterial activity against erythromycin resistant bacteria (for example, resistant pneumococci, streptococci, and mycoplasmas), against which sufficient antibacterial activity cannot be obtained with conventional macrolide antibiotics, and can use as a medicament.

## Claims

1. A compound represented by the formula [1]: 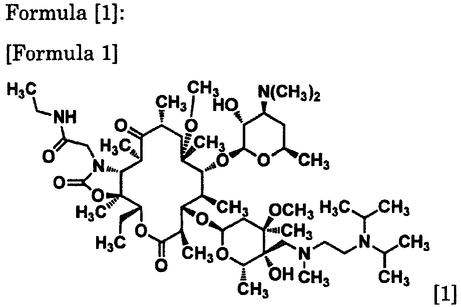 or a pharmaceutically acceptable salt thereof, or a hydrate or a solvate thereof.

2. A pharmaceutical composition containing a compound selected from the group consisting of the compound, a pharmaceutically acceptable salt thereof, and a hydrate and a solvate thereof according to claim 1.

3. An antibacterial agent containing a compound selected from the group consisting of the compound, a pharmaceutically acceptable salt thereof, and a hydrate and a solvate thereof according to claim 1.
